# EUROPEAN PATENT SPECIFICATION

(11) **EP 4 349 335 B1**
(45) Date of publication and mention of the grant of the patent: **09.12.2026**
(21) Application number: 22815205.4
(22) Date of filing: 30.05.2022
(51) Int. Cl.: A61K 31/47, A61L 2/16, A61P 31/20

(54) **5-NITRO-8-HYDROXYQUINOLINE FOR USE IN TREATING A HPV INFECTION**
5-NITRO-8-HYDROXYCHINOLIN ZUR BEHANDLUNG VON HPV INFEKTIONEN
5-NITRO-8-HYDROXYQUINOLÉINE POUR LE TRAITEMENT DES INFECTIONS DE HPV

(30) Priority: 31.05.2021 CN 202110601777
(43) Date of publication of application: 10.04.2024
(73) Proprietor: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: DENG, Yijun, Taizhou City, Jiangsu 225316 (CN)
(74) Representative: Regimbeau
(86) International application number: PCT/CN2022/095861
(87) International publication number: WO 2022/253159

(56) References cited:
- WO-A1-2020/063824
- WO-A1-2021/023231
- CN-A- 103 588 660
- CN-A- 111 514 142
- CN-A- 111 773 193
- CRUZ-GREGORIO ALFREDO ET AL: "Redox-sensitive signalling pathways regulated by human papillomavirus in HPV-related cancers", vol. 31, no. 6, 11 March 2021 (2021-03-11), GB, XP093259706, ISSN: 1052-9276, Retrieved from the Internet <URL:https://onlinelibrary.wiley.com/doi/full-xml/10.1002/rmv.2230> DOI: 10.1002/rmv.2230
- VAN HAU TRUONG ET AL: "Repurposing of Nitroxoline Drug for the Prevention of Neurodegeneration", CHEMICAL RESEARCH IN TOXICOLOGY, vol. 32, no. 11, 22 October 2019 (2019-10-22), US, pages 2182 - 2191, XP093259714, ISSN: 0893-228X, DOI: 10.1021/acs.chemrestox.9b00183
- ROHDE, W. ET AL.: "Hydroxyquinolines inhibit ribonucleic acid-dependent deoxyribonucleic acid polymerase and inactivate Rous sarcoma virus and herpes simplex virus", ANTIMICROBIAL AGENTS AND CHEMOTHERAPY, vol. 10, no. 2, 31 August 1976 (1976-08-31), XP008050819, ISSN: 0066-4804

## Description

### FIELD OF THE INVENTION

The present application relates to use of 5-nitro-8-hydroxyquinoline, a pharmaceutically acceptable salt thereof, a solvate thereof or a prodrug thereof for use for preventing or treating an infection caused by human papillomavirus; wherein the prodrug is (S)-(5-nitroquinolin-8-yloxy)methyl 1-isopropionylpyrrolidine-2-carboxylate: a pharmaceutical composition comprising said 5-nitro-S-hydroxyquinoline as defined previously for use for treating an infection caused by human papillomavirus.

### BACKGROUND OF THE INVENTION

Human papillomavirus (HPV) is a DNA virus belonging to the family *Papillomaviridae,* and it is a very common virus worldwide. HPV members have mucosal epithelial and skin epithelial tropism. In addition to a variety of reproductive tract diseases such as genital warts, they can also cause a variety of malignant tumors, including cervical cancer, penile cancer, anal cancer, oral cancer, pharyngeal and laryngeal cancer, tonsil cancer, esophageal cancer, etc. There are more than 100 types of human papillomavirus, among which at least a dozen types can cause cancer, making it the most important type of human tumor virus. The virus has high infection rate and strong pathogenicity, and it is very harmful to the population, especially women, has attracted extensive attention from domestic and international medical communities. At present, clinically, there are prophylactic multivalent vaccines (HPV6, 11, 16, 18, etc.) that can prevent infection with multiple types of virus, but the use of prophylactic vaccines for infected patients has no significant therapeutic effect. The development of effective drugs that target multiple types of HPV can provide new methods for the clinical treatment and prevention of HPV-related diseases, including malignant tumors.

At present, effective drugs against HPV are still needed in the field.

CRUZ-GREGORIO ALFREDO ET AL: "Redox-sensitive signaling pathways regulated by human papillomavirus in HPV-related cancers", Reviews in medical virology, 31(6), 2021 describes the redox-sensitive signaling pathway regulated by human papillomavirus (HPV) and points out that therapeutic methods for reducing reactive oxygen species (ROS) levels are effective in eliminating HPV infections.

VAN HAU TRUONG ET AL: "Repurposing of Nitroxoline Drug for the Prevention of Neurodegeneration", Chemical research in toxicology, 32(11), 2019, 2182-2191 indicates that nitroxoline is for the prevention of neurodegenerative diseases due to its antioxidant capacity.

CN103588660 A describes acyl aniline compounds (e.g., 5-acetamido-8-hydroxyquinoline) for the treatment of human papillomavirus (HPV) infections.

### SUMMARY OF THE INVENTION

The technical problem to be addressed in the present disclosure is to provide 5-nitro-8-hydroxyquinoline, a pharmaceutically acceptable salt thereof, a solvate thereof or a prodrug thereof for use in the prevention or treatment of an infection caused by human papillomavirus, wherein the prodrug is (S)-(5-nitroquinolin-8-yloxy)methyl 1-isopropionylpyrrolidine-2-carboxylate.

The invention is set out in the appended set of claims.

Throughout the present description, references to methods of treatment by therapy, surgery or in vivo diagnosis are to be interpreted as references to compounds, pharmaceutical compositions and medicaments of the present invention for use in those methods.

### DETAILED DESCRIPTION OF THE INVENTION

### 5-Nitro-8-hydroxyquinoline and use thereof

Nitroxoline has the chemical name of 5-nitro-8-hydroxyquinoline and the CAS number 4008-48-4.

In some embodiments, provided is 5-nitro-8-hydroxyquinoline, a pharmaceutically acceptable salt thereof, a solvate thereof or a prodrug thereof, for use the prevention or treatment of human papillomavirus infection wherein the prodrug is (S)-(5-nitroquinolin-8-yloxy)methyl 1-isopropionylpyrrolidine-2-carboxylate.

In some embodiments, 5-nitro-8-hydroxyquinoline is provided in the form of a pharmaceutically acceptable salt thereof. The pharmaceutically acceptable salt can be an acid addition salt or a base addition salt. In some embodiments, the acid can be an inorganic acid, including but not limited to hydrochloric acid, sulfuric acid, phosphoric acid, hydrobromic acid; or it can be an organic acid, including but not limited to citric acid, maleic acid, oxalic acid, formic acid, acetic acid, propionic acid, valeric acid, glycolic acid, benzoic acid, fumaric acid, trifluoroacetic acid, succinic acid, tartaric acid, lactic acid, glutamic acid, aspartic acid, salicylic acid, pyruvate, methanesulfonic acid, benzenesulfonic acid, p-toluenesulfonic acid. In some embodiments, the base can be an inorganic base, including but not limited to sodium hydroxide, potassium hydroxide, magnesium hydroxide, calcium hydroxide; or it can be an organic base, including but not limited to ammonium hydroxide, triethylamine, N,N-dibenzylethylenediamine, chloroprocaine, choline, ammonia, diethanolamine and other hydroxyalkyl amines, ethylenediamine, N-methylglucosamine, procaine, N-benzylphenethylamine, arginine or lysine; or it can be an alkali metal salt, including but not limited to lithium, potassium and sodium salts (e.g., sodium dihydrogen phosphate and disodium hydrogen phosphate); or it can be an alkaline earth metal salt, including but not limited to barium, calcium and magnesium salts; or it can be a transition metal salt, including but not limited to zinc salt.

The term "pharmaceutically acceptable" means when used in the preparation of a pharmaceutical composition, the pharmaceutical composition is generally safe and nontoxic, meets the biological needs, and can be accepted and served as an agent for a mammal use (e.g., a human).

In other embodiments, 5-nitro-8-hydroxyquinoline is provided in the form of a solvate thereof.

The term "solvate" refers to a substance formed by 5-nitro-8-hydroxyquinoline and a suitable solvent. In particular embodiments, the solvent is water. In other particular embodiments, the solvent is a pharmaceutically acceptable organic solvent.

**In** some other embodiments, 5-nitro-8-hydroxyquinoline is provided in the form of a prodrug thereof, wherein the prodrug is (S)-(5-nitroquinolin-8-yloxy)methyl 1-isopropionylpyrrolidine-2-carboxylate.. The term "prodrug" refers to a derivative of 5-nitro-8-hydroxyquinoline comprising a biological reaction functional group, such that the biological reaction functional group may cleave from the derivative or otherwise react under biological conditions (*in vitro* or *in vivo*) to provide 5-nitro-8-hydroxyquinoline.

The prodrug of 5-nitro-8-hydroxyquinoline is (S)-(5-nitroquinolin-8-yloxy)methyl 1-isopropionylpyrrolidine-2-carboxylate, which has the following structural formula:

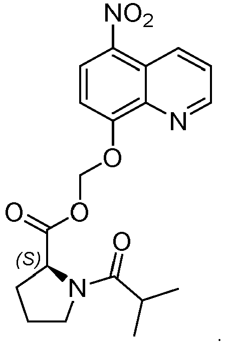

(S)-(5-nitroquinolin-8-yloxy)methyl 1-isopropionylpyrrolidine-2-carboxylate can be prepared by the preparation method of Example 20 of WO2020/063824.

In some embodiments, the prodrug of 5-nitro-8-hydroxyquinoline can be formulated in accordance with the method disclosed in WO 2021/238978.

In the present disclosure, an antiviral drug refers to any compound or composition capable of inhibiting (or reducing, impairing, disturbing, inactivating, killing) the activity, viability, replication, proliferation, growth, infectivity, or virulence of virus *in vivo* or *in vitro.*

In the present disclosure, the antiviral drug is used to prevent, treat, ameliorate a virus-related disease or symptom in a subject.

In the present disclosure, the antiviral drug is used to prevent, treat, ameliorate a human papillomavirus-related disease or symptom in a subject.

Human papillomavirus (HPV) belongs to the genus *Alphapapillomavirus* of the family *Papovaviridae.* HPV is a spherical DNA virus. At present, more than 130 different types of HPV have been isolated, which can be classified into the following types according to the affected tissues and sites:
(1) Skin low-risk type, HPV1, 2, 3, 4, 7, 10, 12, 15, etc.;
(2) Skin high-risk type, HPV5, 8, 14, 17, 20, 36, 38, etc.;
(3) Mucosa low-risk type, HPV6, 11, 13, 32, 34, 40, 42, 43, 44, 54, etc.;
(4) Mucosa high-risk type, HPV16, 18, 30, 31, 33, 35, 53, 39, etc.

In some embodiments, the antiviral drug of the present disclosure is for use for mucosa type (high-risk and low-risk) human papillomavirus-related diseases or symptoms; mention can be made of HPV6, 11, 13, 16, 18, 30, 31, 32, 33, 34, 35, 39, 40, 42, 43, 44, 53, 54.

In some particular embodiments, the HPV is notably selected from the group consisting of: human papillomavirus 6 (HPV6), human papillomavirus 11 (HPV11), human papillomavirus 16 (HPV16), human papillomavirus 18 (HPV18) or a combination thereof.

In the present disclosure, when referred to "the compound of the present disclosure", it refers to 5-nitro-8-hydroxyquinoline, a pharmaceutically acceptable salt thereof a solvate thereof or a prodrug thereof, wherein the prodrug is (S)-(5-nitroquinolin-8-yloxy)methyl 1-isopropionylpyrrolidine-2-carboxylate.

### Method for prevention or treatment

The present disclosure provides a method for preventing or treating a viral infection in a subject.

In some embodiments, the method for preventing a viral infection in a subject comprises, or consists of, the following step: administering a prophylactically effective amount of 5-nitro-8-hydroxyquinoline, a pharmaceutically acceptable salt thereof a solvate thereof or a prodrug thereof, to a subject wherein the prodrug is (S)-(5-nitroquinolin-8-yloxy)methyl 1-isopropionylpyrrolidine-2-carboxylate.

In some embodiments, the method for treating a viral infection in a subject comprises, or consists of, the following step: administering a therapeutically effective amount of 5-nitro-8-hydroxyquinoline, a pharmaceutically acceptable salt thereof a solvate thereof or a prodrug thereof, to a subject, wherein the prodrug is (S)-(5-nitroquinolin-8-yloxy)methyl 1-isopropionylpyrrolidine-2-carboxylate.

"Administering" or "administration", when applied to an animal, a human, a subject, a cell, a tissue, an organ or a biological fluid, refers to contacting an exogenous drug (such as the compound of the present disclosure), a therapeutic agent or a composition with the animal, human, subject, cell, tissue, organ or biological fluid.

The term "subject" refers to a mammal, including, for example, camel, donkey, zebra, cattle, pig, horse, goat, sheep, cat, dog, rat, rabbit, guinea pig, mouse, primate.

In some particular embodiments, the subject is a human.

In some particular embodiments, the subject is exposed to, is susceptible to, is suspected to have, or has already affected with virus infection.

"Already affected" should be understood in the broadest way, also including that the probability of having a viral infection, at a given significance threshold, is statistically higher than that of controls. For statistically "significant", *p* is set to, for example, but not limited to 0.5, 0.1, 0.05, 0.01, 0.005, 0.001, 0.0005, 0.0001, or even lower. For example, when a comparison is made between two individuals or groups, and the resulting*p*-value is lower than a given specific *p*-value, the two individuals or groups would be considered to have a statistically significant difference.

In the method for prevention, the subject is susceptible to, potentially exposed to, or exposed to a viral infection.

In the method for treatment, the subject is suspected of having or has already affected with viral infection.

The term "treating" or "treatment" refers to eliminating the disease, preventing disease progression, slowing disease progression, reducing the duration of one or more symptoms associated with the disease, improving or reversing at least one measurable parameter associated with the disease, or increasing the survival of subjects with the disease.

The term "preventing" or "prevention" refers to a prophylactic measure against a pathogenic factor, such as exposure to virus, and can be measures taken for an environment or for a subject. In particular embodiments, preventing or prevention is to use the compound or pharmaceutical composition of the present disclosure to treat a subject, an environment or an object that has not been infected, so as to prevent infection, reduce the risk of infection, reduce the probability of infection, delay the occurrence or onset of a symptom of infection, etc.

The term "effective amount" refers to an amount of the compound or pharmaceutical composition of the present disclosure that elicits the desired effect in a subject, an environment or an object.

In some embodiments, "therapeutically effective amount" is an amount sufficient to treat a disease state or symptom, especially which associated with viral infection state, or an amount to block, delay or reverse the viral infection or other undesirable symptoms.

In some embodiments, "prophylactically effective amount" is an amount that will have a predetermined prophylactic effect when administered to a subject, an environment or an object.

The therapeutic or prophylactic effect does not necessarily occur once one dose is given, but may occur after a series of doses have been given.

In particular embodiments, those skilled in the art can determine the selection of an effective amount based on consideration of a variety of factors (e.g., through clinical trials), the factors include the activity of the compound of the present disclosure, the metabolic rate of the specific compound used, the disease to be treated, the symptoms involved, the route of administration, the time of administration, the severity of the disease, the patient's body weight, the patient's immune status, other drugs (compounds and/or materials) used in combination with the specific compound used, and other factors known to those skilled in the art. Those skilled in the art can easily determine the required effective amount. The effective amount can be obtained from the dose-response curve derived from an animal model testing system, and it is allowed to be determined according to the opinion of a physician and the situation of each patient. Wherein, the correlation between animal and human doses is described in Freireich et al., 1966, Cancer Chemother Rep 50:219, and the surface area of human body can be approximately determined by the height and body weight of the patient.

In general, the appropriate daily dose of the compound of the present disclosure will be the amount of compound that effectively exerts a prophylactic or therapeutic effect.

The effective amount of the compound of the present disclosure can be 0.01 mg/kg to 500 mg/kg, preferably 1 mg/kg to 200 mg/kg, more preferably 10 mg/kg to 100 mg/kg; specifically, mention may be made of 0.01, 0.05, 0.1, 0.5, 1, 2, 3, 4, 5, 6, 7, 8, 9, 10, 20, 30, 40, 50, 60, 70, 80, 90, 100, 150, 200, 250, 300, 350, 400, 450, 500 mg/kg, and the ranges between any two of the above values.

If necessary, the daily dose of the compound of the present disclosure can be separately administered throughout the day in two, three, four, five, six or more subdoses at appropriate time intervals; optionally, the subdose is a dose in unit dosage form.

The pharmaceutical composition or compound provided by the present disclosure can be administered in accordance with conventional clinical treatment methods, for example, for viral infections, it can be directly administered by perfusion or application to the infected site; the dose used for administration can be the dose determined by the healthcare practitioner.

In particular embodiments, the pharmaceutical composition or compound provided by present disclosure is prepared into a dosage form for vaginal administration.

Although the compound of the present disclosure can be administered alone, it is also allowed to administer the compounds in the form of a pharmaceutical composition.

The term "unit dose" as used in the present disclosure refers to a physically discrete unit suitable for administration to a subject, an environment or an object as a unit dose. Each unit contains a predetermined amount of the compound or a composition thereof of the present disclosure.

In some embodiments, the unit dose described in the present disclosure is expressed by volume and is selected from the group consisting of 0.1 ml, 0.15 ml, 0.2 ml, 0.5 ml, 1.0 ml, 1.5 ml, 2.0 ml, 2.5 ml, 3.0 ml, 4.0 ml, 5.0 ml, 10.0 ml, 20.0 ml, 30.0 ml, 40.0 ml, 50.0 ml, 60.0 ml, 70.0 ml, 80.0 ml, 90.0 ml, 100.0 ml, 150 ml, 200 ml, 250.00 ml, and ranges between any two of the above values.

Those skilled in the art understand that a unit dose that is too large or too small results in clinical inconvenience. For example, when administered vaginally to a human subject, the unit dose is preferably in the range of 1 ml to 100 ml, for example, 1, 10, 20, 30, 40, 50, 60, 70, 80, 90, 100 ml, and the ranges between any two of the above values. When administered subcutaneously to a human subject, the unit dose is preferably in the range of 0.5 ml to 1.0 ml. When administered intravenously to a human subject, the unit dose is preferably in the range of 30.0 ml to 1000 ml.

It should be understood here that although the unit dose is expressed as volume, this does not mean that the compound or a composition thereof of the present disclosure shall only be formulated a liquid formulation. When prepared into a solid (powder or lyophilized powder) formulation, the volume of a unit dose represents the volume of reconstituted liquid prepared from the powder or lyophilized powder.

In some embodiments, the compound or a composition thereof of the present disclosure is administered to the subject at the following frequency: 1 to 4 times every 4 years, 1 to 3 times every 3 years , 1 to 2 times every 2 years, 1 time a year, 2 times a year, 3 times a year, 4 times a year, 5 times a year, 6 times a year, 1 time a month, 2 times a month, 3 times a month, 4 times a month, 5 times a month, 6 times a month, 7 times a month, 8 times a month, 1 time a week, 2 times a week, 3 times a week, 4 times a week, 5 times a week, 6 times a week, 1 time every three days, 2 times every three days, 3 times every three days, 1 time every two days, 2 times every two days, 1 time a day, 2 times a day.

In some embodiments, the frequency of administration of the compound or a composition thereof of the present disclosure can be the same or different.

In some embodiments, the time interval between each administration is the same or different.

In some embodiments, the administration of the compound or a composition thereof of the present disclosure is systemic or topical.

In some embodiments, the compound or a composition thereof of the present disclosure is administered by parenteral (such as intramuscular, intraperitoneal, intravenous, subcutaneous, intradermal) injection.

In some embodiments, the compound or a composition thereof of the present disclosure is administered intramuscularly.

In other embodiments, the compound or a composition thereof of the present disclosure is delivered transdermally (for example, in a manner that does not disrupt the epithelial cell barrier by mechanical devices).

In some other embodiments, the compound or a composition thereof of the present disclosure is administered through rectal or vaginal route.

### Pharmaceutical composition

According to some embodiments, provided is a pharmaceutical composition comprising the compound of the present disclosure and optionally a pharmaceutically acceptable excipient. Suitable pharmaceutically acceptable excipients are described in Remington Pharmaceutical Sciences by E.W. Martin.

In particular embodiments, the pharmaceutical composition comprises one or more pharmaceutically acceptable excipients, including diluent, excipient, filler, binder, wetting agent, disintegrant, absorption enhancer, surfactant, adsorption carrier, lubricant or synergist, etc. conventional in the pharmaceutical field.

Those skilled in the art are able to understand that when the pharmaceutical composition is in the form of powder or lyophilized powder, the difference between a pharmaceutical composition in powder form and a pharmaceutical composition in liquid form lies in water content and/or buffer environment.

In particular embodiments, the pharmaceutical composition of the present disclosure can be prepared into the form of injection, suppository, tablet, pill, capsule, suspension, emulsion, spray, disinfectant, lotion for administration.

In some embodiments, the pharmaceutical composition can be in a form suitable for oral administration, for example, tablet, lozenge, aqueous or oil suspension, dispersible powder or granule, emulsion, capsule, or syrup. The oral composition can be prepared according to any method known in the art for preparing a pharmaceutical composition, and such compositions can contain one or more ingredients selected from the group consisting of sweetener, flavoring agent, coloring agent and preservative. The excipient suitable for preparing a tablet can be an inert excipient, such as calcium carbonate, sodium carbonate, lactose, calcium phosphate or sodium phosphate; a granulating and disintegrating agent, for example, microcrystalline cellulose, croscarmellose sodium, corn starch or alginic acid; a binder, for example, starch, gelatin, polyvinylpyrrolidone or arabic gum; and a lubricant, for example, magnesium stearate, stearic acid or talc.

In some embodiments, the aqueous suspension comprises the compound of the present disclosure and an excipient suitable for the preparation of an aqueous suspension. Such excipients are suspending agents, for example, sodium carboxymethylcellulose, methylcellulose, hydroxypropylmethylcellulose, sodium alginate, polyvinylpyrrolidone and arabic gum; dispersing or wetting agents, which can be a naturally occurring phospholipid, for example, lecithin, polyoxyethylene stearate, or a condensation product of ethylene oxide with long chain fatty alcohol, polyethylene oxide sorbitol monooleate, or polyethylene oxide sorbitan monooleate. The water suspension can also contain a preservative (for example, ethylparaben or propylparaben), a colorant, a flavoring agent.

In some embodiments, the oil suspension can be formulated by suspending an active ingredient in a vegetable oil such as peanut oil, olive oil, sesame oil or coconut oil, or mineral oil. The oil suspension can contain a thickening agent, for example beeswax, hard paraffin or cetyl alcohol.

The pharmaceutical composition of the present disclosure can also be in the form of oil-in-water emulsion. The oil phase can be vegetable oil, for example, olive oil or peanut oil, or mineral oil. A suitable emulsifier can be a naturally occurring phospholipid, for example, soy lecithin. Such preparations can also contain buffers, preservatives, coloring agents and antioxidants.

The pharmaceutical composition of the present disclosure can be in the form of sterile injectable aqueous solution. Acceptable solvents that can be used are water, Ringer's solution and isotonic sodium chloride solution. An injection solution or microemulsion can be injected into the bloodstream of a patient by local bolus injection. Or, the solution and microemulsion are preferably administered in a manner that maintains a constant circulating concentration of the compound of the present disclosure. To maintain this constant concentration, a continuous intravenous drug delivery device can be used.

In some particular embodiments, provided is a suppository form for vaginal or rectal administration. These pharmaceutical compositions can be prepared by mixing the compound of the present disclosure with a non-irritating excipient (solid at room temperature, but liquid in the vagina or rectum). Such compositions comprise cocoa butter, glycerin gelatin, hydrogenated vegetable oil, polyethylene glycols with various molecular weights, or fatty acid esters of polyethylene glycol.

In the present specification, when describing a numerical range, the expressions "...to...", "within the range of..." or "between the range of..." are used to include the endpoint values. This expression is an abbreviation in which each integer or decimal within the range is considered unambiguously indicated.

In the present specification, when describing numerical values, unless otherwise specified, statistical errors and errors introduced by operations shall be taken into consideration. The extent of error depends on the specific situation. For example, as for the amount of administration, errors due to weighing operation and errors introduced in the formulation process and the like are involved, for example, 150 ml can be deemed as 150 ml ± 10%.

Unless otherwise specified, the articles "a", "an", "the" as used herein are intended to include "at least one" or "one or more".

The present disclosure is further illustrated below by way of examples, but it is not thereby limited to the scope of these examples. The experimental methods, without specifying particular conditions in the examples, follow conventional methods and conditions, or follow the methods and conditions suggested in product instruction.

In the following Effect Examples, the names and Latin names of each virus are as follows:
Human Papillomavirus 6 (HPV6);
Human Papillomavirus 11 (HPV11);
Human Papillomavirus 16 (HPV16);
Human Papillomavirus 18 (HPV18);
Novel coronavirus (SARS-CoV-2);
Chikungunya virus (CHIKV);
Dengue Virus 2;
Influenza A H1N1;
Middle East Respiratory Syndrome Coronavirus (MERS-CoV);
Rift Valley Fever Virus (RVFV);
Tacaribe Virus (TCRV);
wt-Cottontail Rabbit Papillomavirus (wt-CRPV);
mE8-Cottontail Rabbit Papillomavirus (mE8-CRPV);
Murine Papillomavirus (MmuPV1).

### Example 1. Preparation of 5-nitro-8-hydroxyquinoline

The 5-nitro-8-hydroxyquinoline in the present disclosure is not limited by the preparation method, it is obtained according to any preparation method disclosed in prior art, or commercially available products can be used.

### Example 2. Preparation of the prodrug of 5-nitro-8-hydroxyquinoline

According to the method disclosed in Example 20 of WO2020/063824, the prodrug of 5-nitro-8-hydroxyquinoline, (S)-(5-nitroquinolin-8-yloxy) methyl 1-isopropionylpyrrolidine-2-carboxylate, was prepared.

### Effect Example 1

The antiviral activity of 5-nitro-8-hydroxyquinoline against human papillomavirus (HPV) was tested and evaluated using transient transfection assays.

This evaluation system included plasmids expressing viral helicase E1 and replication origin binding protein E2, plasmids containing NanoLuc gene, the expression of which was driven by a TK promoter (Promega), as well as replication origin sequences of human papillomavirus strains listed below. Compared with using E1 or E2 alone, the synergistic binding and helicase activity of E1 and E2 expressed through the plasmids drove the replication of the reporter plasmid, resulting in an increase in NanoLuc activity of more than 100 folds. The positions of replication origin sequences of the three NanoLuc reporter plasmids were 7825-7993:1-99, 7662-7901:1-99, and 7740-7837:1-102 for HPV11 (NCBI Accession No. HE611260.1), HPV16 (KP212151.1), and HPV18 (KC470230.1), respectively. The positions of E1 open reading frame (ORF) sequences corresponding to HPV11, HPV16 and HPV18 were located at 832-2781, 864-2813 and 914-2887 in the same sequences, respectively. The positions of E2 open reading frame (ORF) sequences corresponding to HPV11, HPV16 and HPV18 were located at 2723-3826, 2755-3852 and 2817-3908 in the same sequences, respectively. The tests on HPV6 were done by using similar test methods to those on HPV11, HPV16, and HPV18.

The cells used for transient transfection were C-33A cells (ATCC HTB-31) in DMEM medium supplemented with Earle's salt, L-glutamine and 2% fetal bovine serum. In the experiment, the NanoLuc expression plasmid (5 ng/well) was co-transfected with the E1 and E2 expression plasmids of the homologous virus by using Lipofectamine LTX and PlusReagent (Invitrogen, Thermo Fisher Scientific). After 1 hour of incubation, the transfected cells were inoculated into wells containing 5-fold diluted test drug and the positive control drug 9-(2-phosphonomylmethoxyethoxy) guanine (PMEG) (Sigma-Aldrich). The transfected cells were also inoculated into duplicated dishes containing the same dilution of test drug to assess cytotoxicity. All transfected monolayers of cells were incubated at 37°C for 48 hours. The NanoLuc activity expressed by reporter plasmids was detected with Nano-Glo reagent (Promega), the cell viability in cytotoxicity plates was measured with CellTiter-Glo reagent (Promega), and the luminescence intensity was quantified on a microplate reader. The concentrations of the compound that reduced the number of reporter plasmid copies by 50% (EC₅₀), 90% (EC₉₀) were identified from the experimental data.

The detailed methods were as described in references (Beadle, J. et al. Synthesis and Antiviral Evaluation of Octadecyloxyethyl Benzyl 9-[(2-Phosphonomethoxy) ethyl] guanine (ODE-Bn-PMEG), a Potent Inhibitor of Transient HPV DNA Amplification. J Med Chem. 2016, 59(23): 10470-8; Kachaeva, M. et al. In vitro activity of novel 1,3-oxazole derivatives against HPV. Ibnosina J Med Biomed Sci. 2017; 9(4): 111-8).

The detailed test results were shown in Tables 1 and 2. The results showed that the antiviral activity of 5-nitro-8-hydroxyquinoline was better than that of the positive control drug.

### Effect Example 2

The antiviral activity of 5-nitro-8-hydroxyquinoline against Human Papillomavirus 11, Human Papillomavirus 16, Human Papillomavirus 18, Human Papillomavirus 6 was tested by using a method similar to that used in Effect Example 1, and the test results were shown in Table 2.

When the SI₅₀ value > 10, the compound was determined to be active against the virus. The results showed that 5-nitro-8-hydroxyquinoline had specific inhibitory activity against HPV, while this activity was not observed against other viruses.

### Effect Example 3 (prodrug)

According to the method of Test Example 4 in WO 2021/238978, the prodrug prepared in Example 2 was administered to animals (dogs), and its conversion to the active form 5-nitro-8-hydroxyquinoline was detected in circulating samples. Thus, the prodrug and 5-nitro-8-hydroxyquinoline were equivalent in anti-HPV effect.

**Table 1. Data for activity of the positive control drug against HPV**

| No. | Virus name | Virus strain | Cell line | Positive control drug (9-[2-(phosphonomethoxy)ethyl]guanine) | | |
|---|---|---|---|---|---|---|
| | | | | Concentration range | EC_{50/}µM | EC₉₀/µM |
| 1 | *HPV11* | HE611260.1 | C-33 A | 0.048-150 µM | 1.03 | 4.47 |
| 2 | *HPV18* | KC470230.1 | C-33 A | 0.048-150 µM | 0.63 | >150.00 |
| 3 | *HPV6* | FR751330.1 | C-33 A | 0.048-150 µM | 0.56 | 6.35 |
| 4 | *HPV16* | KP212151.1 | C-33 A | 0.001-250 µM | 0.28 | 2.3 |

**Table 2. Data for the activity of 5-nitro-8-hydroxyquinoline against the listed viruses**

| | Strain | Cell line | Concentration of 5-nitro-8-hydroxyquinoline | EC₅₀ | EC₉₀ | CC₅₀ | SI₅₀* | SI₉₀ |
|---|---|---|---|---|---|---|---|---|
| Human Papillomavirus 11 | HE611260.1 | C-33 A | 0.048-150 µM | 1.04 | 2.99 | 55.49 | 53 | 19 |
| Human Papillomavirus 16 | KP212151.1 | C-33 A | 0.048-150 µM | 2.82 | 5.11 | 66.33 | 23 | 13 |
| Human Papillomavirus 18 | KC470230.1 | C-33 A | 0.048-150 µM | 1.01 | 3.99 | 64.96 | 64 | 16 |
| Human Papillomavirus 6 | FR751330.1 | C-33 A | 0.048-150 µM | 0.86 | 5.41 | 47.84 | 55 | 9 |
| Human Papillomavirus 16 | KP212151.1 | C-33 A | 0.001-250 µM | 0.26 | 0.54 | 77.33 | 297 | 143 |

| | | | | | | | | |
|---|---|---|---|---|---|---|---|---|
| * SI₅₀ = CC₅₀/EC₅₀. | | | | | | | | |

## Claims

1. 5-nitro-8-hydroxyquinoline, a pharmaceutically acceptable salt thereof, a solvate thereof or a prodrug thereof for use for preventing or treating an infection caused by human papillomavirus; wherein the prodrug is (S)-(5-nitroquinolin-8-yloxy)methyl 1-isopropionylpyrrolidine-2-carboxylate.

2. The 5-nitro-8-hydroxyquinoline, the pharmaceutically acceptable salt thereof, the solvate thereof or the prodrug thereof for use according to claim 1, wherein the 5-nitro-8-hydroxyquinoline, the pharmaceutically acceptable salt thereof, the solvate thereof or the prodrug thereof is for use in a mammal.

3. The 5-nitro-S-hydroxyquinoline, the pharmaceutically acceptable salt thereof, the solvate thereof or the prodrug thereof for use according to claim 1 or 2, wherein the virus is selected from the group consisting of: human papillomavirus 6 (HPV6), human papillomavirus 11 (HPV11), human papillomavirus 16 (HPV16), human papillomavirus 18 (HPV18) or a combination thereof.

4. The 5-nitro-S-hydroxyquinoline, the pharmaceutically acceptable salt thereof, the solvate thereof or the prodrug thereof for use according to claim 2, wherein:
the mammal is human.

5. A pharmaceutical composition for use in treating a viral infection, the pharmaceutical composition comprises:
- a pharmaceutically acceptable excipient, and
- any one selected from the following: 5-nitro-8-hydroxyquinoline, a pharmaceutically acceptable salt thereof, a solvate thereof or a prodrug thereof;
the virus is human papillomavirus;
the prodrug is (S)-(5-nitroquinolin-8-yloxy)methyl 1-isopropionylpyrrolidine-2-carboxylate.

6. The pharmaceutical composition for use according to claim 5, wherein the virus is selected from the group consisting of: human papillomavirus 6 (HPV6), human papillomavirus 11 (HPV11), human papillomavirus 16 (HPV16), human papillomavirus 18 (HPV18) or a combination thereof

## Patentansprüche

1. 5-Nitro-8-hydroxychinolin, ein pharmazeutisch annehmbares Salz davon, ein Solvat davon oder ein Prodrug davon zur Verwendung zur Prävention oder Behandlung einer Infektion, die von humanem Papillomavirus verursacht wird; wobei das Prodrug (S)-(5-Nitrochinolin-8-yloxy)methyl-1-isopropionylpyrrolidin-2-carboxylat ist.

2. 5-Nitro-8-hydroxychinolin, das pharmazeutisch annehmbare Salz davon, das Solvat davon oder das Prodrug davon zur Verwendung nach Anspruch 1, wobei das 5-Nitro-8-hydroxychinolin, das pharmazeutisch annehmbare Salz davon, das Solvat davon oder das Prodrug davon zur Verwendung bei einem Säugetier ist.

3. 5-Nitro-8-hydroxychinolin, das pharmazeutisch annehmbare Salz davon, das Solvat davon oder das Prodrug davon zur Verwendung nach Anspruch 1 oder 2, wobei das Virus ausgewählt ist aus der Gruppe bestehend aus: humanem Papillomavirus 6 (HPV6), humanem Papillomavirus 11 (HPV11), humanem Papillomavirus 16 (HPV16), humanem Papillomavirus 18 (HPV18) oder einer Kombination davon.

4. 5-Nitro-8-hydroxychinolin, das pharmazeutisch annehmbare Salz davon, das Solvat davon oder das Prodrug davon zur Verwendung nach Anspruch 2, wobei:
das Säugetier menschlich ist.

5. Pharmazeutische Zusammensetzung zur Verwendung bei der Behandlung einer Virusinfektion, wobei die pharmazeutische Zusammensetzung umfasst:
- einen pharmazeutisch annehmbaren Hilfsstoff, und
- ein beliebiges, aus den folgenden ausgewähltes: 5-Nitro-8-hydroxychinolin, ein pharmazeutisch annehmbares Salz davon, ein Solvat davon oder ein Prodrug davon;
wobei das Virus humanes Papillomavirus ist;
wobei das Prodrug (S)-(5-Nitrochinolin-8-yloxy)methyl-1-isopropionylpyrrolidin-2-carboxylat ist.

6. Pharmazeutische Zusammensetzung zur Verwendung nach Anspruch 5, wobei das Virus ausgewählt ist aus der Gruppe bestehend aus: humanem Papillomavirus 6 (HPV6), humanem Papillomavirus 11 (HPV11), humanem Papillomavirus 16 (HPV16), humanem Papillomavirus 18 (HPV18) oder einer Kombination davon.

## Revendications

1. La 5-nitro-8-hydroxyquinoléine, un sel pharmaceutiquement acceptable de celle-ci, un solvate de celle-ci ou un promédicament de celle-ci, pour son utilisation dans la prévention ou le traitement d'une infection causée par le papillomavirus humain, dans laquelle le promédicament est le (S)-(5-nitroquinolin-8-yloxy)méthyl 1-isopropionylpyrrolidine-2-carboxylate.

2. La 5-nitro-8-hydroxyquinoléine, le sel pharmaceutiquement acceptable de celle-ci, le solvate de celle-ci ou le promédicament de celle-ci destinés à être utilisés selon la revendication 1, dans laquelle la 5-nitro-8-hydroxyquinoléine, le sel pharmaceutiquement acceptable de celle-ci, le solvate de celle-ci ou le promédicament de celle-ci sont destinés à être utilisés chez un mammifère.

3. La 5-nitro-8-hydroxyquinoléine, le sel pharmaceutiquement acceptable de celle-ci, le solvate de celle-ci ou le promédicament de celle-ci destinés à être utilisés selon la revendication 1 ou 2, dans laquelle le virus est choisi dans le groupe constitué de : le papillomavirus humain 6 (HPV6), le papillomavirus humain 11 (HPV11), le papillomavirus humain 16 (HPV16), le papillomavirus humain 18 (HPV18), ou une combinaison de ceux-ci.

4. La 5-nitro-8-hydroxyquinoléine, le sel pharmaceutiquement acceptable de celle-ci, le solvate de celle-ci ou le promédicament de celle-ci destinés à être utilisés selon la revendication 2, dans laquelle : le mammifère est un être humain.

5. Composition pharmaceutique pour son utilisation dans le traitement d'une infection virale, ladite composition pharmaceutique comprenant :
- un excipient pharmaceutiquement acceptable ; et
- un composé choisi parmi les suivants : la 5-nitro-8-hydroxyquinoléine, un sel pharmaceutiquement acceptable de celle-ci, un solvate de celle-ci, ou un promédicament de celle-ci;
dans laquelle :
le virus est un papillomavirus humain ; et
le promédicament est le (S)-(5-nitroquinolin-8-yloxy)méthyl 1-isopropionylpyrrolidine-2-carboxylate.

6. Composition pharmaceutique destinée à être utilisée selon la revendication 5, dans laquelle le virus est choisi dans le groupe constitué de : papillomavirus humain 6 (HPV6), papillomavirus humain 11 (HPV11), papillomavirus humain 16 (HPV16), papillomavirus humain 18 (HPV18), ou une combinaison de ceux-ci.
